(19) Europäisches Patentamt

European Patent Office

Office européen des brevets

(11) Veröffentlichungsnummer: **0 406 711 A1**

## (12) EUROPÄISCHE PATENTANMELDUNG

(21) Anmeldenummer: 90112410.7

(22) Anmeldetag: 29.06.90

(51) Int. Cl.5: **C12N 9/56, C12N 1/38**

(30) Priorität: 01.07.89 DE 3921764

(43) Veröffentlichungstag der Anmeldung:
09.01.91 Patentblatt 91/02

(84) Benannte Vertragsstaaten:
**AT DE DK**

(71) Anmelder: **FORSCHUNGSZENTRUM JÜLICH GMBH**
**Wilhelm-Johnen-Strasse**
**D-5170 Jülich(DE)**

(72) Erfinder: **Giesecke, Eberhard Ulrich**
**Martinusstrasse 10**
**D-5170 Jülich(DE)**
Erfinder: **Bierbaum, Gabriele, Dr.**
**Welldorfer Strasse 8**
**D-5170 Jülich(DE)**
Erfinder: **Wandrey, Christian, Prof. Dr.**
**Wolfshovener Strasse 139**
**D-5170 Jülich(DE)**

(54) Verfahren zur Gewinnung von alkalischer Serin-Protease durch ammoniumlimitierte Fermentation von Bacillus-Stämmen.

㉗ Zur Gewinnung von Subtilisin werden Bakterien des Genus Bacillus in einem Fermenter auf synthetischem Medium kultiviert, wobei in einer ersten Anwachsphase mit einer $NH_4$-Anfangskonzentration von 0,5 bis 150 mM, insbesondere 1 bis 50 mM und Nachdosierung der C-Quelle für die Aufrechterhaltung ihrer Konzentration im Bereich von 100 mM bis 1 M bis zum Absinken der Ammoniumkonzentration unter einen N-limitierenden Wert in der Kultur zwischen 0,05 und 2 mM, insbesondere 0,1 und 0,3 mM gearbeitet wird, wonach der N-limitierende Wert der Ammoniumkonzentration aufrechterhalten und der Gehalt der C-Quelle zwischen 10 mM und 2 M eingestellt wird bis zum Ende der Produktionsphase, wonach das gebildete Subtilisin abgesondert wird. Als C-Quelle werden insbesondere Glycerin, Acetat oder Hexosen verwendet. Vorzugsweise wird die Biomassekonzentration im Medium durch Nachfüttern von C-Quelle erhöht. Ein Sauerstoffpartialdruck über der Kultur von > 11,5 Pa ist zweckmäßig. Als Proteaseproduzent dient insbesondere Bacillus licheniformis DSM 1969.

## VERFAHREN ZUR GEWINNUNG VON ALKALISCHER SERIN-PROTEASE DURCH AMMONIUMLIMITIERTE FERMENTATION VON BACILLUS-STÄMMEN

Die Erfindung bezieht sich auf ein Verfahren zur Gewinnung von alkalischer Serin-Protease (Subtilisin) in einem Fermenter durch Bakterienkultur mit Ammoniumlimitierung unter Verwendung eines Stammes des Genus Bacillus.

Subtilisin ist die wirtschaftlich bedeutenste Protease und wird hauptsächlich in Waschmitteln eingesetzt. Das Enzym wird in industriellem Maßstab durch Fermentation von Bacillus-Stämmen, insbesondere Bacillus licheniformis unter Verwendung von komplexen Medien gewonnen, wie z. B. präzipitiertem Casein, Stärkehydrolysat und Sojamehl in einer bestimmten ausgewogenen Zusammensetzung (US-PS 3,740,318).

In Anbetracht der wirtschaftlichen Bedeutung gibt es seit geraumer Zeit zahlreiche Untersuchungen, die sich mit den Bedingungen für die Proteasebildung befassen:

So werden bereits von A T. Bull (J. appl. Biotechnol. 22 (1972) 261-92) die Zusammensetzung des Mediums, der pH-Wert, Temperatur, Rührgeschwindigkeit, Belüftung, spezielle Zusätze, Erntezeitpunkt und genetische Stabilität als Einflußgrößen genannt. F. G. Heineken u. a. (J. of Gen. Microbiol. 73 (1972) 35-44) befassen sich mit dem Einfluß von Ammonium und Glucose auf die Exoenzymbildung und berichten über die Reprimierung der Bildung durch Ammonium. Verschiedene Autoren (z. B. J. Chaloupka u. a., Can. J. Microbiol. 28 (1982) 1214-18) beschreiben die Reprimierung der Enzymbildung durch Aminosäurezugabe bzw. die Förderung der Proteaseproduktion durch Ammoniummangel, wobei von G.W. Hanlon u. a. im J. Gen. Microbiol 128 (1982) 845-51 über die Förderung der Proteaseproduktion durch Ammoniumerschöpfung (gearbeitet wurde mit Ansätzen, die 2,3 mM $NH_4Cl$ enthielten) berichtet wird.

Nach J. Frankena u. a. (Appl. Microbiol. Biotechnol. 22 (1985) 169-176) wird eine maximale Proteaseproduktion in glucoselimitierter Chemostatkultur durch Wachstumsraten um $\mu = 0,22$ $h^{-1}$ erreicht. J.T.M. Wouters u. a. (FEMS Lett. 1 (1977) 109-112) berichten über die Proteaseproduktion bei N-limitiertem Wachstum (auf Glutamat).

D. h., die zahlreichen Untersuchungen über den Einfluß eines Glucosemangels, Ammoniummangels, Mangel an bestimmten Amminosäuren, (geringer) Wachstumsraten oder die speziell anzustrebende Wachstumsphase (insbesondere Ankopplung an die Sporulation) ergeben kein klares Bild der zu favorisierenden Produktionsbedingungen.

Es wurde nun überraschenderweise gefunden, daß eine ganz erhebliche Steigerung der Proteasebildung in rein synthetischen Medien (d. h. Medien, die keine Bioextrakte undefinierter Zusammensetzung enthalten) unter Einhaltung einer bestimmten Ammoniumlimitierung erzielt werden kann.

Demgemäß ist das erfindungsgemäße Verfahren der eingangs genannten Art dadurch gekennzeichnet, daß man die Bakterien auf einem synthetischen Medium kultiviert, wobei in einer ersten Anwachsphase mit einer NH-Anfangskonzentration von 0,5 bis 150 mM, insbesondere 1 bis 50 mM und Nachdosierung der C-Quelle für die Aufrechterhaltung ihrer Konzentration im Bereich von 100 mM bis 1 M bis zum Absinken der Ammoniumkonzentration unter einen N-limitierenden Wert in der Kultur zwischen 0,05 und 2 mM, insbesondere 0,1 und 0,3 mM gearbeitet wird, wonach der N-limitierende Wert der Ammoniumkonzentration aufrechterhalten und der Gehalt der C-Quelle zwischen 10 mM und 2 M eingestellt wird bis zum Ende der Produktionsphase, wonach das gebildete Subtilisin abgesondert wird.

Auf diese Weise ist eine Gewinnung von Subtilisin möglich, das in überraschend hoher Konzentration [z. Zt. in Mengen bis ca. 200 PE/ml (Kulturüberstand) ] anfällt. Aus dem rein synthetischen Medium kann die Protease nach Abtrennung der Bakterien durch Mikrofiltration durch Ultrafiltration in farbloser Qualität gewonnen werden, die für den Einsatz in flüssigen Waschmitteln gut geeignet ist.

Als C-Quelle dienen dabei insbesondere Glycerin, Acetat oder Hexosen und als Stickstoffquelle Ammoniumsalze. Zweckmäßigerweise wird die Biomassekonzentration im Medium durch Nachfüttern der C-Quelle erhöht und damit die Proteasebildung beschleunigt.

Die Einhaltung des genannten Ammoniumgehalts in der Kultur kann durch wiederkehrende Messung und Zudosierung erreicht werden, wobei jeweils im Zeitpunkt der Zugabe kurzzeitig aus dem gewünschten Bereich auslaufende Spitzenwerte nicht stören. Vorzugsweise wird der Ammoniumgehalt jedoch kontinuierlich überwacht und durch entsprechende Nachdosierung im gewünschten engen Konzentrationsbereich gehalten. Dabei kann in der ersten Anwachsphase vorzugsweise mit einer noch nicht limitierenden Ammoniumkonzentration gearbeitet werden.

Der gelöste Sauerstoff im Medium soll innerhalb eines Bereichs entsprechend 2,3 - 115 Pa Sauerstoffpartialdruck (1 - 50 % Sauerstoffsättigung), vorzugsweise 11,5 - 23 Pa (5 -10 % Sauerstoffsättigung) liegen. Die Konzentrationen der C-Quelle wird in der Anwachsphase der Kultur zwischen 100 mM und 1 M, vorzugsweise bis zu 600 mM und insbesondere bei ca. 200 mM gewählt, da bei zu hohen Konzentrationen ein verzögertes Anwachsen der Kultur beobachtet wird. Während der Kultur wird dann durch Nachfüttern der C-Quelle auf Konzentrationen in der Kultur zwischen 10 mM und 2 M - insbesondere über 50 mM - für eine erhöhte Biomassekonzentration gesorgt. Dabei soll keine Limitierung der C-Quelle eintreten, wodurch die Proteaseausschüttung nachhaltig gehemmt würde. Besonders wenn höhere Sauerstoffpartialdrücke gewählt werden, steigt der Verbrauch der Kultur an C-Quelle, so daß sich ihre Kontrolle (z. B. des Glycerin- oder Glucosegehaltes) während der Fermentation empfiehlt.

Folgende Salze sind außerdem zweckmäßigerweise im Medium vorhanden:
15 - 25 mM $KPO_4$, 1 - 5 mM $MgSO_4$, 10 - 500 mM $CaCl_2$, 40 - 100 $\mu$M $FeSO_4$, 40 - 50 $\mu$M $MnSO_4$.
Das vorzugsweise eingesetzte Standardmedium ist in Beispiel (s. u.) aufgeführt. Die Fermentationstemperatur liegt zweckmäßigerweise zwischen 35 und 45 °C, vorzugsweise bei 37 - 39 °C. Der Fermentations-pH wird im allgemeinen zwischen pH 6 und 8, vorzugsweise bei pH 7 gehalten.

Als Proteaseproduzent hat sich insbesondere Bacillus licheniformis, insbesondere Bacillus licheniformis DSM 1969 bewährt.

Nachfolgend wird die Erfindung anhand eines Beispiels näher erläutert, in dem die kontinuierliche Überwachung und Steuerung des Ammoniumgehaltes beschrieben wird:

Zur Gewinnung der Protease wurde Bacillus licheniformis DSM 1969 in einem Glycerin-Medium bei 37 °C zur Vorkultur bis zu einer optischen Dichte (OD 600 nm) zwischen 3 und 5 im Schüttelkolben (200 ml Medium in einem 1-l-Kolben mit 4 Schikanen) und anschließend bei 37 °C im Fermenter (4-l-Medium) in einem Fedbatch-Versuch gezüchtet. Als Standardmedium wurde ein Glycerin-Medium folgender Zusammensetzung verwendet:
200 mM Glycerin
12 mM $(NH_4)_2SO_4$ (nur Fermentation)
25 mM $(NH_4)_2SO_4$ (nur Schüttelkolben)
10 mM $K_2HPO_4$
10 mM $KH_2PO_4$
1 mM $MgSO_4$
500 $\mu$M $CaCl_2$
100 $\mu$M $FeSO_4$
50 $\mu$M $MnSO_4$
1 g/l Polypropylenglycol 1200 (nur Fermentation)
50 mM Tris-hydroxymethyl-aminomethan (nur Schüttelkolben)
50 mM Maleinsäure (nur Schüttelkolben) PH 7
Es wurden folgende Fermentationsbedingungen eingehalten:
Die Temperatur betrug 37 °C in der Vorkultur und 37 °C im Fermenter. Der Sauerstoffpartialdruck wurde mindestens bei 11,5 Pa gehalten. (Dieser Wert entspricht 5 % der möglichen Sauerstoffsättigung. Während der Anwachsphase wurde mit 300 Upm gerührt. Sobald der Partialdruck 11,5 Pa erreicht war, wurde dieser Wert durch eine Drehzahlregulierung aufrechterhalten. Bei einer Belüftung von 1 vvm und 100 mbar Überdruck konnten Drehzahlen bis zu maximal 1000 Upm erreicht werden.) Die Vorkultur wurde mit 100 rpm im Kreisschüttler geschüttelt.

Der pH-Wert der Kultur wurde durch automatische Titration bei pH 7,0 gehalten.

Der Ammoniumgehalt des Fermentationsmediums wurde durch eine gassensitive Ammoniumelektrode kontinuierlich überwacht. Zu Beginn war die Ammoniumkonzentration 16 mM.

Nach Absinken des Ammoniumgehalts auf Konzentrationen unter 0,15 mM wurde mittels einer adaptiven Regelung über eine Pumpe Ammonium zudosiert. Als Ammoniumsollwert war 0,15 mM Ammonium vorgegeben.

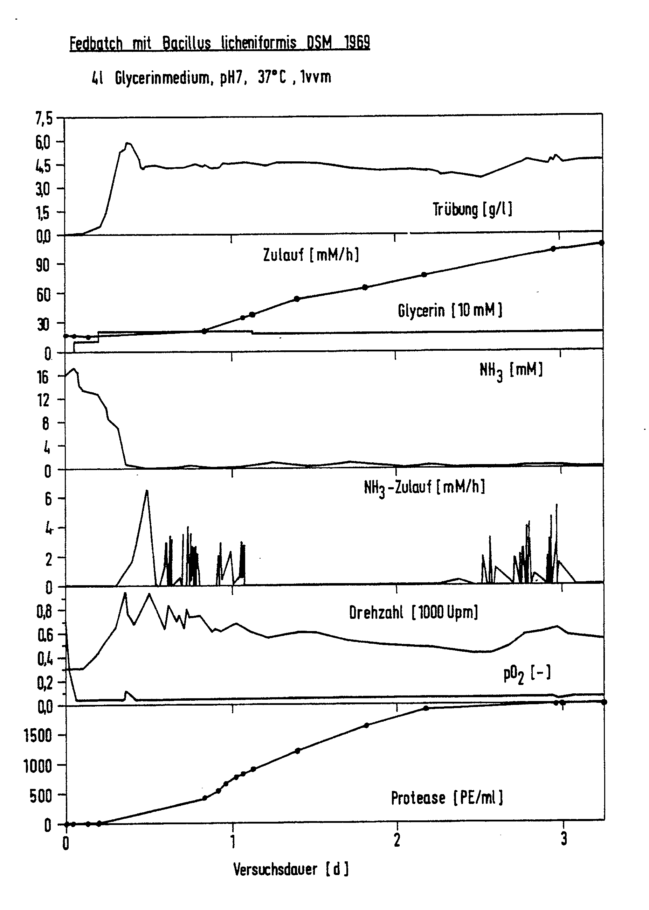

Der Glyceringehalt des Fermentationsmediums wurde durch diskontinuierliche Probennahme überwacht. Nach Anwachsen der Kultur wurde Glycerin über eine Pumpe zudosiert. In der beigefügten Abbildung ist der Verlauf eines typischen Versuches dargestellt. Von oben nach unten ist das Wachstum der Biomasse (Trübung), Konzentration und Zudosierung der C-Quelle Glycerin und der N-Quelle Ammonium, die Regulation der Belüftung und die Ausschüttung der Protease zu erkennen.

Als das exponentielle Wachstum der Kultur einsetzte (d.h. bei Absinken des Sauerstoffpartialdruckes auf 5 % Sauerstoffsättigung)) wurde Glycerin zudosiert, so daß in der ersten Phase der Fermentation ein Sollwert von 150 - 200 mM Glycerin vorlag. Im weiteren Verlauf der Fermentation wurde der Glyceringehalt auf 1 M erhöht. Es wurden ungefähr 5 g Biotrockenmasse erzeugt. Nach Verbrauch des Ammoniums schaltete sich die Ammoniumpumpe ein und hielt eine Ammoniumkonzentration von 0,15 mM im Medium ein. Kurz darauf begann die Ausschüttung der Protease. Der Sauerstoffpartialdruck wurde wie oben beschrieben geregelt, und es wurden maximale Drehzahlen von 950 Upm erreicht. In diesem Versuch wurden 1985 Proteaseeinheiten pro ml (getestet mit dem Substrat N-Carbobenzoxy-glycin-p-nitrophenylester; eine Proteaseeinheit pro ml setzt bei 25 °C, pH 6,67 und einer Anfangskonzentration von 6,055 mM CBZ-glycin-p-nitrophenylester 1,65 nmol/ml*min p-Nitrophenol frei) produziert.

Der Kulturüberstand (aus einem anderen Versuch) konnte durch Ultrafiltration (Amicon-Membran YM 10) auf 25000 Proteaseeinheiten pro ml aufkonzentriert werden.

## Ansprüche

1. Verfahren zur Gewinnung von alkalischer Serin-Protease (Subtilisin) in einem Fermenter durch Bakterienkultur mit Ammoniumlimitierung unter Verwendung eines Stammes des Genus Bacillus, **dadurch gekennzeichnet,** daß man die Bakterien auf einem synthetischen Medium kultiviert, wobei in einer ersten Anwachsphase mit einer $NH_4$-Anfangskonzentration von 0,5 bis 150 mM, insbesondere 1 bis 50 mM und Nachdosierung der C-Quelle für die Aufrechterhaltung ihrer Konzentration im Bereich von 100 mM bis 1 M bis zum Absinken der Ammoniumkonzentration unter einen N-limitierenden Wert in der Kultur zwischen 0,05 und 2 mM, insbesondere 0,1 und 0,3 mM gearbeitet wird, wonach der N-limitierende Wert der Ammoniumkonzentration aufrechterhalten und der Gehalt der C-Quelle zwischen 10 mM und 2 M eingestellt wird bis zum Ende der Produktionsphase, wonach das gebildete Subtilisin abgesondert wird.

2. Verfahren nach Anspruch 1,
**dadurch gekennzeichnet,**
daß das synthetische Medium als C-Quelle Glycerin, Acetat oder Hexosen enthält.

3. Verfahren nach Anspruch 1 oder 2,
**dadurch gekennzeichnet,**
daß die Biomassekonzentration im Medium durch Nachfüttern der C-Quelle für die Aufrechterhaltung eines Konzentrationsbereichs von 50 mM bis 2 M erhöht wird.

4. Verfahren nach einem der vorangehenden Ansprüche,
**dadurch gekennzeichnet,**
daß der Sauerstoffpartialdruck über der Kultur bei > 11,5 Pa gehalten wird.

5. Verfahren nach einem der vorangehenden Ansprüche,
**dadurch gekennzeichnet,**
daß der Ammoniumgehalt in der Kultur durch kontinuierliche Überwachung und Nachdosierung eingehalten wird.

6. Verfahren nach einem der vorangehenden Ansprüche,
**dadurch gekennzeichnet,**
daß als Proteaseproduzent Bacillus licheniformis verwendet wird.

7. Verfahren nach Anspruch 6,
**dadurch gekennzeichnet,**
daß als Proteaseproduzent Bacillus licheniformis DSM 1969 verwendet wird.

8. Verfahren nach einem der vorangehenden Ansprüche,
**dadurch gekennzeichnet,**
daß das Enzym von der Kulturflüssigkeit durch Ultrafiltration abgesondert wird.

Fedbatch mit Bacillus licheniformis DSM 1969

4l Glycerinmedium, pH7, 37°C , 1vvm

7,5

6,0
4,5
3,0
1,5                                    Trübung [g/l]
0,0

                        Zulauf [mM/h]
90
60                                     Glycerin [10 mM]
30
0

16                                     NH₃ [mM]
12
8
4
0

                        NH₃ -Zulauf [mM/h]
6
4
2
0

0,8                     Drehzahl [1000 Upm]
0,6
0,4                                    pO₂ [-]
0,2
0,0

1500
1000
500                                    Protease [PE/ml]
0

0          1          2          3

Versuchsdauer [d]

Europäisches
Patentamt

**EUROPÄISCHER RECHERCHENBERICHT**

Nummer der Anmeldung

EP 90 11 2410

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, Band 97, 1982, Seite 497, Zusammenfassung Nr. 70766f, Columbus, Ohio, US; T.A. CHERDYNTSEVA et al.: "Bacillus subtilis var. amyloliquefaciens and its biosynthesis of extracellular protease with coagulase activity and produced under conditions of a medium with a limited nitrogen source", & MIKROBIOLOGIYA 1982, 51(3), 431-5 * Zusammenfassung * | | $C\ 12\ N\quad 9/56$<br>$C\ 12\ N\quad 1/38$ |
| D,A | JOURNAL OF GENERAL MICROBIOLOGY, Band 128, 1982, Seiten 845-851; G.W. HANLON et al.: "The influcence of glucose, ammonium and magnesium availability on the production of protease and bacitracin by Bacillus licheniformis" * Ganzes Artikel * | | |
| A | CHEMICAL ABSTRACTS, Band 95, 1981, Seiten 320-321, Zusammenfassung Nr. 146864t, Columbus, Ohio, US; G.W. HANLON et al.: "Bacitracin and protease production in relation to sporulation during exponential growth of Bacillus licheniformis on poorly utilized carbon and nitrogen sources", & J. BACTERIOL. 1981, 147(2), 427-31 * Zusammenfassung * | | **RECHERCHIERTE SACHGEBIETE (Int. Cl.5)**<br><br>$C\ 12\ N$ |
| A | J. CHEM. TECH. BIOTECHNOL., Band 41, 1988, Seiten 197-206; M.M. KOLE et al.: "Production of protease by Bacillus subtilis using simultaneous control of glucose and ammonium concentrations" * Ganzes Artikel * | | |

Der vorliegende Recherchenbericht wurde für alle Patentansprüche erstellt

| Recherchenort | Abschlußdatum der Recherche | Prüfer |
|---|---|---|
| DEN HAAG | 05-10-1990 | HUBER A. |